# EUROPÄISCHE PATENTSCHRIFT

(11) **EP 0 568 872 B1**
(45) Veröffentlichungstag und Bekanntmachung des Hinweises auf die Patenterteilung: **27.12.1996**
(21) Anmeldenummer: 93106595.7
(22) Anmeldetag: 23.04.1993
(51) Int. Cl.: C07C 45/00, C07C 49/17

(54) **Verfahren zur Herstellung von Dihydroxyaceton**
Process for the preparation of dihydroxyacetone
Procédé pour la préparation de dihydroxyacétone

(30) Priorität: 04.05.1992 DE 4214808
(43) Veröffentlichungstag der Anmeldung: 10.11.1993
(73) Patentinhaber: BASF Aktiengesellschaft, 67063 Ludwigshafen (DE)
(72) Erfinder: Gehrer, Eugen, Dr., W-6700 Ludwigshafen (DE); Harder, Wolfgang, Dr., W-6940 Weinheim (DE); Vogel, Herbert, Dr., W-6700 Ludwigshafen (DE); Knuth, Bernhard, Dr., 67059 Ludwigshafen (DE); Ebel, Klaus, Dr., W-6700 Ludwigshafen (DE); Groening, Carsten, Dr., W-6700 Ludwigshafen (DE)

(56) Entgegenhaltungen:
- EP-A- 0 410 613
- PATENT ABSTRACTS OF JAPAN vol. 16, no. 358 (C-970)1992 & JP-A-41 12 847 (NIPPON DASEI CHEM CO LTD)

## Beschreibung

Die vorliegende Erfindung betrifft ein Verfahren zur Herstellung von Dihydroxyaceton durch die Thiazoliumylid-katalysierte Kondensation von Formaldehyd.

Die Thiazoliumylid-katalysierte Formoin-Kondensation von Formaldehyd zu Dihydroxyaceton (DHA) in einem wasserfreien Medium wurde erstmals von Castells (Tetrahedron Lett. 21, 4517 (1980)) beschrieben.

Matsumoto et. al. (J. Am. Chem. Soc. 106, 4829 (1984)) untersuchten diese Reaktion systematisch in verschiedenen Lösungsmitteln und fanden bei der Verwendung wasserfreier Lösungsmittel, wie Ethanol, Butanol, Dioxan, Diethylenglykol, Dimethylether, Ethylpropionat, Dimethylsulfoxid, Dimethylformamid und Heptan gute Ergebnisse. Lediglich bei der Verwendung von Wasser war die Ausbeute an DHA gleich Null.

Gemäß EP-A 306 215 muß die Thiazoliumylid-katalysierte Umsetzung von Formaldehyd zu DHA in einem im wesentlichen wasserfreien Reaktionsmedium vorgenommen werden, wobei unter diesem Begriff Wassergehalte von weniger als 0,4 Gew.-%, vorzugsweise von weniger als 0,1 Gew.-% verstanden werden. Aus diesem Grunde wird nach diesem Verfahren auch trockener, wasserfreier Formaldehyd als Edukt eingesetzt.

Gemäß EP-A 410 613 können katalytisch aktive Thiazoliumylide auch aus Thiazoliumsalzen erzeugt werden, indem man die entsprechenden Thiazoliumsalze in einem organischen Lösungsmittel löst und aus der organischen Lösung deren Anionen mittels Wasser extrahiert. Nach beendeteter Extraktion wird die wäßrige Phase von der organischen abgetrennt. Die im organischen Medium gelösten Restmengen Wasser werden anschließend mit einem Teil der organischen Lösungsmittel azeotrop abdestilliert. Die Kondensation des in der organischen Phase gelösten wasserfreien Formaldehyds zu DHA setzt dabei erst ein, sobald der Wassergehalt des Reaktionsmediums auf 0,1 Gew.-% gesunken ist.

Ursache für die schlechten Ergebnisse der genannten Umsetzung in Gegenwart von Wasser ist vermutlich der Umstand, daß in wäßrigem Medium befindliche Hydroxylionen, die als Katalysator dienenden Thiazoliumverbindungen in 2-Position des Thiazoliumringes nukleophil angreifen und dort nicht nur das katalytisch aktive Zentrum der Katalysatorverbindung blockieren, sondern letztendlich die Ringöffnung und irreversible Zerstörung der Katalysatorverbindung bewirken.

JP-A 112847/1992 betrifft ein Verfahren zur Herstellung von 1,3-Dihydroxyaceton durch die Kondensation von Formaldehyd mittels eines an ein Polystyrolharz gebundenen quarternären Thiazoliumsalzkatalysators in wäßrigem Medium.

Formaldehyd wird bei praktisch allen industriellen Herstellungsverfahren als wäßrige Lösung gewonnen. Die Gewinnung von wasserfreiem Formaldehyd, etwa in Form von getrockneten Lösungen von Paraformaldehyd oder gasförmigem Formaldehyd, ist mit einem erheblichen technischen Mehraufwand verbunden, der die Wirtschaftlichkeit von Verfahren zur Herstellung von DHA und insbesondere von dessen Folgeprodukten Glycerin (EP-A 306 215) und 1,2-Propandiol (deutsche Patentanmeldung Anmelde-Nr. P 41 38 792.9) stark beeinträchtigt. Der vorliegenden Erfindung lag daher die Aufgabe zugrunde, ein Verfahren zu finden, mit dem es möglich ist, aus rohen wäßrigen Formaldehydlösungen ohne weitere Aufarbeitung mittels Thiazoliumylid-Katalysatoren DHA herzustellen.

Dementsprechend wurde ein Verfahren zur Herstellung von Dihydroxyaceton durch die Thiazoliumylid-katalysierte Kondensation von Formaldehyd gefunden, das dadurch gekennzeichnet ist, daß man die Umsetzung in einem Reaktionssystem, bestehend aus einer wäßrigen und mindestens einer flüssigen organischen Phase und bei einem pH-Wert der wäßrigen Phase von weniger als 8 ausführt.

Bei dem erfindungsgemäßen Verfahren wird somit ein Reaktionssystem angewandt, das aus mindestens einer organischen, nicht mit Wasser mischbaren, flüssigen Phase und einer wäßrigen Phase besteht. Dabei befindet sich der Katalysator, beispielsweise ein Thiazoliumylid, im wesentlichen in der organischen Phase, wohingegen der zu kondensierende Formaldehyd, sich vorzugsweise in der wäßrigen Phase löst. Die beim erfindungsgemäßen Verfahren ablaufenden chemischen Vorgänge lassen sich vereinfacht so beschreiben, daß der Formaldehyd möglicherweise unter Mitwirkung des verwendeten organischen Lösungsmittels aus der wäßrigen Phase in die organische Phase transportiert wird und dort, von dem im wesentlichen in der organischen Phase gelösten Katalysator zu DHA kondensiert wird, welches anschließend in die wäßrige Phase migriert, aus der es isoliert werden kann. Das erfindungsgemäße Verfahren findet somit unter Phasentransferkatalyse statt.

Zur Durchführung des erfindungsgemäßen Verfahrens wird der Formaldehyd im allgemeinen in Form seiner wäßrigen Lösung eingesetzt. Es kann der Formaldehyd jedoch auch in anderer Form, beispielsweise als Formaldehyd/Wasserdampf-Gasgemisch zugeführt werden.

Die Katalysatoren für die Kondensation des Formaldehyds befinden sich erfindungsgemäß im wesentlichen in der nicht mit Wasser mischbaren, organischen Phase. Diese nicht mit Wasser mischbare, flüssige organische Phase ist im allgemeinen ein organisches Lösungsmittel, es kann aber auch eine auf einem organischen oder anorganischen Trägermaterial aufgezogene und fest an diesem anhaftende, flüssige, organische Phase sein, in der der Katalysator gelöst ist. Weiterhin kann der Katalysator selbst als organische Phase verwendet werden, beispielsweise in Form seiner Schmelze.

Als organische Lösungsmittel sind im erfindungsgemäßen Verfahren eine Vielzahl von nicht mit Wasser mischbaren, organischen Lösungsmitteln geeignet, beispielsweise höhere Alkohole, wasserunlösliche N,N-Dialkylformamide, wasserunlösliche N-Alkylpyrrolidone, Kohlenwasserstoffe oder halogenierte Kohlenwasserstoffe. Besonders bevorzugt werden höhere Alkohole, insbesondere C₅-C₃₀-Alkohole sowie wasserunlösliche N,N-Dialkylformamide, wie N,N-Dipropylformamid, N,N-Dibutylformamid, N,N-Dipentylformamid usw. verwendet. Besonders vorteilhafte alkoholische Lösungsmittel sind C₆-C₂₂-Alkohole, wie n-Hexanol, Cyclohexanol, 2-Ethylhexanol, n-Octanol, Nonanole, Decanole, Dodecanole, Hexadecanole und Eicosanole, von denen wiederum solche Alkohole aufgrund ihrer guten technischen Zugänglichkeit bevorzugt eingesetzt werden, die sich von den natürlich vorkommenden Fettsäuren ableiten oder wie 2-Ethylhexanol für andere technische Verwendungen in großen Mengen produziert werden und daher kostengünstig zur Verfügung stehen. Desgleichen können mit gutem Erfolg Mischungen verschiedener, wasserunlöslicher organischer Lösungsmittel verwendet werden.

Trägergestützte flüssige organische Phasen (supported-liquid phase) können beispielsweise hergestellt werden, indem man ein Trägermaterial großer Oberfläche mit einer Lösung des Katalysators in einem lipophilen organischen Lösungsmittel, gegebenenfalls unter Zusatz eines leicht flüchtigen Hilfslösungsmittels aufbringt. Dazu können all die Techniken angewandt werden, wie sie zur Präparation von Trägermaterialien für die Gaschromatographie gang und gäbe sind.

Werden lipophile Katalysatoren eingesetzt, die z.B. mit langkettigen lipophilen Resten, z.B. langkettigen Alkylresten, substituiert sind, so können diese Katalysatoren sowohl in An- als auch in Abwesenheit eines organischen Lösungsmittels selbst die flüssige organische Phase ausbilden.

Im allgemeinen und vorzugsweise werden aber organische Lösungsmittel als organische Phase im erfindungsgemäßen Verfahren eingesetzt, wobei in der Regel ein Volumenverhältnis wäßrige Phase/organische Phase von 0,01 bis 5, vorzugsweise von 0,1 bis 1 und besonders bevorzugt von 0,2 bis 0,5 eingestellt wird. Die Anwendung größerer Wassermengen ist ebenfalls möglich, doch führt dies im allgemeinen nicht zu weiteren Vorteilen.

Als Katalysatoren werden im erfindungsgemäßen Verfahren zweckmäßigerweise Thiazoliumring-haltige Katalysatoren verwendet, die in 2-Stellung des Thiazoliumrings unsubstituiert sind, beispielsweise Thiazoliumylide der Formel I 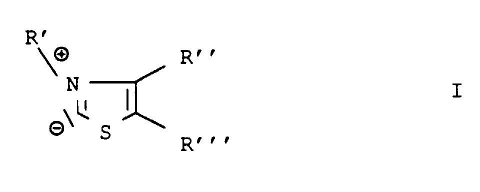 Isoliert man diese Ylide, so erhält man je nach der Art der Erzeugung der Thiazoliumylide Dimere der Formel Ia 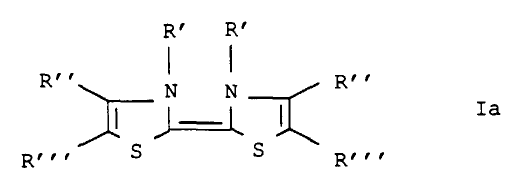 oder Alkoholaddukte der Formel Ib

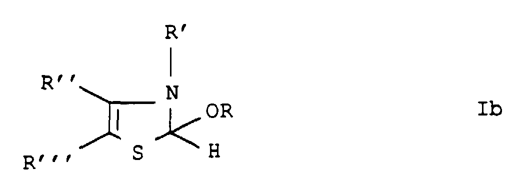

Die Verbindungen der Formel Ia lassen sich vorzugsweise aus Thiazoliumylid-Lösungen isolieren, die z.B. durch die Deprotonierung der betreffenden Thiazoliumsalze mittels Aminen erhalten worden sind, wohingegen die Verbindungen Ib, sich vorzugsweise aus Thiazoliumylid-Lösungen gewinnen lassen, die beispielsweise durch Umsetzung von N-Formyl-N-Alkyl-o-mercaptoanilin-Derivaten mit Orthoformiaten erzeugt worden sind. Die Verbindugnen Ia und Ib sind stabil, können kristallin erhalten werden und insbesondere Ib als stabile Lagerform für die Ylide I genutzt werden.

Für die katalytische Aktivität der als Katalysatoren verwendeten Thiazoliumverbindungen ist es allein wichtig, daß die daraus hergestellten Thiazoliumylide in 2-Stellung des Thiazoliumrings unsubstituiert sind. Ansonsten können die Thiazoliumylid-Katalysatoren mit beliebigen Substituenten R', R'' oder R''' substituiert sein. Beispielsweise können Thiazoliumylide der Formel Ic 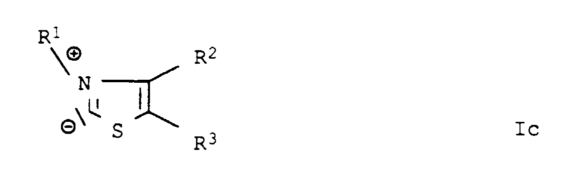 oder Benzothiazoliumylide Id 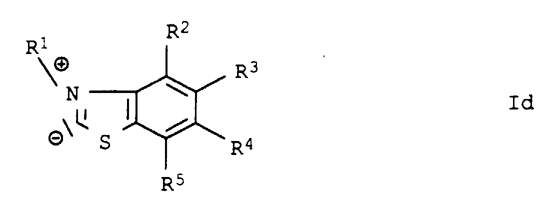 in denen R¹ z.B. für eine C₁-C₃₀-Alkylgruppe, vorzugsweise eine C₂- bis C₂₀-Alkylgruppe oder eine Halogenalkylgruppe entsprechender Kohlenstoffzahl, die als Halogenatome vorzugsweise Fluor- oder Chloratome enthalten kann, eine an einen polymeren Träger gebundene C₁- bis C₃₀-Alkylengruppe, eine C₇- bis C₂₀-Aralkylgruppe, vorzugsweise eine C₇ bis C₁₂-Aralkylgruppe, insbesondere die Benzylgruppe oder eine Arylgruppe, wie die Phenyl- oder Naphthylgruppe, stehen kann. Die Reste R² bis R⁵ können prinzipiell von beliebiger Art sein, soweit sie sich unter den Reaktionsbedingungen inert verhalten. So können die Reste R², R³, R⁴ und R⁵ beispielsweise gleich oder verschieden sein und für Wasserstoff, ein Halogenatom, eine C₁- bis C₂₀-Alkyl- oder Alkoxygruppe, vorzugsweise eine C₁- bis C₄-Alkylgruppe, eine C₁- bis C₂₀-, vorzugsweise eine C₂-bis C₄-Carboxamidogruppe, eine C₇- bis C₁₂-Aralkylgruppe, vorzugsweise die Benzylgruppe und/oder eine Phenyl- und Naphthylgruppe stehen. Die Reste R¹, R², R³, R⁴ und R⁵ können gewünschtenfalls noch unter den Reaktionsbedingungen inerte Substituenten tragen, wie Alkylgruppen, Alkoxygruppen, Halogenatome, C₂- bis C₁₀-Dialkylaminogruppen, C₁- bis C₁₀-Thioethergruppen, Nitrogruppen oder Nitrilogruppen. Da die Herstellung von Thiazoliumsalzen, in denen die Reste R¹, R², R³, R⁴ und R⁵ solche zusätzlichen Substituenten tragen, im allgemeinen ziemlich aufwendig ist, da in der Regel teure Ausgangsmaterialien verwendet werden müssen, ist die Anwendung der daraus hergestellten Thiazolium- oder Benzothiazoliumylide im erfindungsgemäßen Verfahren aus Kostengründen im allgemeinen weniger bevorzugt. Bevorzugt und vorteilhaft werden im erfindungsgemäßen Verfahren insbesondere Thiazoliumylide der Formel Ic sowie mehrkernig-aromatische Thiazoliumylide, wie Benzothiazoliumylide Id oder Naphthathiazoliumylide. Besonders bevorzugt werden die Naphthathiazoliumylide der Formel II 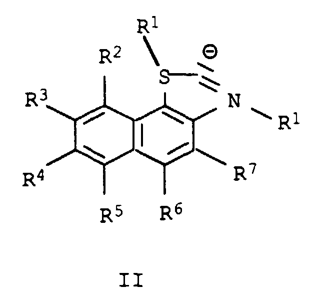 eingesetzt, wobei für deren zusätzlichen Reste R⁶ bis R⁷ die gleichen Maßgaben gelten, wie sie für die Reste R² bis R⁵ oben dargelegt wurden, d.h., die Reste R⁶ bis R⁷ haben die gleiche Bedeutung wie sie für die Reste R² bis R⁵ angegeben wurde. Besonders vorteilhaft werden im erfindungsgemäßen Verfahren die Thiazoliumylide Ic, die Benzothiazoliumylide Id und die Naphthathiazoliumylide II oder deren Vorstufe, die entsprechenden Thiazoliumsalze, mit lipophilen Resten R¹ eingesetzt, insbesondere solche, in denen R¹ ein C₁₀- bis C₂₀-Alkylrest ist. Insbesondere langkettige Reste R¹ können die Lipophilie der Thiazoliumsalze und der daraus hergestellten Thiazoliumylidkatalysatoren und somit deren Löslichkeit im organischen Lösungsmittel des erfindungsgemäß angewandten Zweiphasensystems erhöhen.

Die Thiazolium-, Benzothiazolium- und Naphthathiazoliumylide können auch an einem polymeren, organischen oder anorganischen Träger, beispielsweise einem vernetzten Styrol-Divinylbenzol-Harz oder einem Phenol-Formaldehyd-Harz, z.B. über den Rest R¹ angebunden sein, wobei der Rest R¹ eine C₁- bis C₂₅-, vorzugsweise eine C₁- bis C₄-Alkylengruppe sein kann.

Zur Erzeugung dieser Thiazoliumylidkatalysatoren gibt es verschiedenerlei Möglichkeiten. So können die betreffenden Thiazoliumylide in Lösung durch Erhitzung der inneren, betainartigen Thiazoliumsalze der allgemeinen Formel III 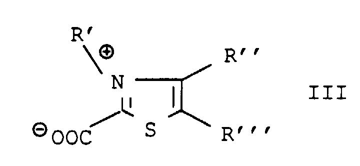 unter Abspaltung von Kohlendioxid erzeugt werden.

Eine andere Möglichkeit besteht in der direkten Synthese von Benzothiazoliumyliden Id durch die Umsetzung der entsprechenden N-Formyl-N-Alkyl-o-mercaptoanilin-derivaten IV mit wasserentziehenden Mitteln, wie Orthoameisensäureestern, gemäß Reaktionsgleichung (1)

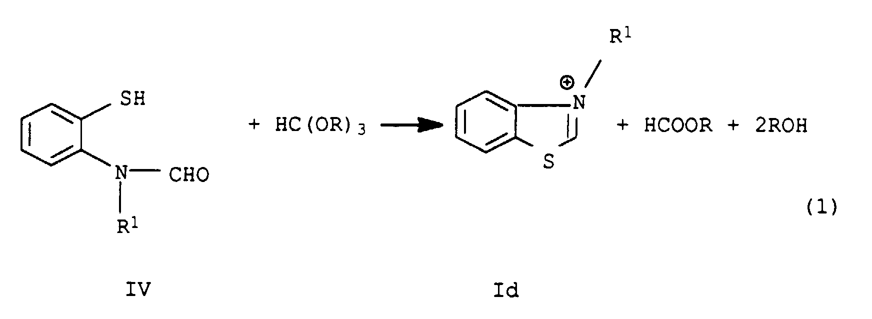

Die Verbindungen IV bilden sich bei der Umsetzung der N-Alkyl-o-mercaptoaniline mit Ameisensäure und deren Derivaten, wie Ameisensäuren und Orthoameisensäureestern. Die als Ausgangsprodukte zur Herstellung dieser Verbindungen dienenden o-Mercaptoaniline können beispielsweise nach dem Verfahren der DE-PS 367 346 erhalten werden. Die Reste R der Orthoameisensäureester sind vorzugsweise C₁- bis C₄-Alkylgruppen.

Eine weitere Möglichkeit zur Erzeugung der Thiazoliumylidkatalysatoren I besteht im Erhitzen der Wasser- oder Alkoholaddukte Ib (R = H oder C₁- bis C₂₀-Alkyl, C₆- bis C₁₂-Aryl) in Lösung, wobei Wasser bzw. der betreffende Alkohol in der Regel bei Temperaturen von 100 bis 250⁰C und zweckmäßigerweise unter dem Eigendruck des Reaktionssystems aus diesen Addukten abgespalten werden.

Eine andere Methode zur Herstellung der Thiazoliumylidkatalysatoren I besteht in der Deprotonierung der entsprechenden Thiazoliumring-haltigen Verbindungen, wie denen der Formel V 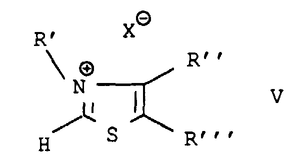 in denen das Gegenanion X^{⊖} zum positiv geladenen Thiazoliumkation beliebig gewählt werden und beispielsweise ein Halogenidanion oder eine Nitrat-, Perchlorat-, Tetrafluoroborat-, Toluolsulfonat-, Methansulfonat-, Benzolsulfonat-, Hydrogensulfat- oder Acetat-Gruppe sein kann, mit Hilfe von Basen.

Solche Thiazoliumsalze sind leicht nach dem allgemein anwendbaren Verfahren von Matsumoto et al. (J.Am.Chem. Soc. 106, 4829 (1984)) durch die Umsetzung der betreffenden Thiazole, Benzothiazole, Phenanthro- und Naphthathiazole mit Alkylhalogeniden erhältlich. Die betreffenden Thiazole können nach bekannten Methoden (s. z.B. G.Vernin, General Synthetic Methods for Thiazole and Thiazolium Salts, in: Thiazole and its Derivatives (The Chemistry of Heterocyclic Compounds, Vol. 34, part I), Ed.: J.V. Metzger, John Wiley, New York (1979)), synthetisiert werden. Die Alkylhalogenide sind leicht aus den entsprechenden Fettalkoholen, z.B. durch deren Behandlung mit Halogenwasserstoffsäuren, zugänglich.

Die Naphthathiazole können z.B. nach dem Verfahren von Wanzlick et al, Liebigs Ann. Chem. 708, 155 (1967) hergestellt werden.

Als Hilfsbasen zur Erzeugung der Thiazoliumylide können z.B. tertiäre Amine mit 3- bis 30 Kohlenstoffatomen oder cyclische Amine, wie sie z.B. in EP-A 219 317 beschrieben sind, eingesetzt werden, die aufgrund ihrer Basenstärke in der Lage sind, die betreffenden Thiazoliumsalze in 2-Stellung des Thiazoliumringes zu deprotonieren.

Geeignete tertiäre Amine sind beispielsweise Trimethylamin, Triethylamin, Tripropylamin, Tributylamin, Ethyldiisopropylamin, Decyldiethylamin, Tridecylamin, Chinuclidin, Diazabicyclo-[2,2,2]-octan, N-Methylpiperidin, N-Ethylpiperidin, N-Propylpiperidin, N-Butylpiperidin, N,N'-Dimethylpiperazin, N-Methylmorpholin, Dimethylbenzylamin, Dibenzylmethylamin, Benzyldioctylamin, Benzyldiethylamin, Cyclohexyldiethylamin, Dicyclohexylmethylamin, Dicyclohexylethylamin usw. Besonders bevorzugt wird Triethylamin verwendet. Von den cyclischen Amidinen werden 1,5-Diazabicyclo-[4,3,0]-non-5-en, 1,8-Diazabicyclo-[5,4,0]-undec-7-en und 1,5,7-Triazabicyclo-[4,4,0]-dec-7-en vorzugsweise eingesetzt.

Es können auch polymere tertiäre Amine als Hilfsbasen verwendet werden, beispielsweise vernetzte Styrol-Divinylbenzol-Harze oder Phenol-Formaldehyd-Harze, die Seitenketten mit tertiären Aminogruppen tragen oder deren Arylgruppen mit Dialkylaminogruppen substituiert sind. Solche polymeren Amine werden üblicherweise als Anionenaustauscher eingesetzt.

Es ist bemerkenswert, daß im erfindungsgemäß angewandten, flüssigen Zweiphasensystem lipophile Thiazoliumsalze, wie Hexadecylbenzothiazoliumsalze, besonders leicht zu den entsprechenden, katalytisch aktiven Thiazoliumyliden deprotoniert werden und daher selbst im sauren pH-Bereich katalytisch aktiv sind. So können auch schwach basische Stickstoffverbindungen, wie Pyridin oder N-Alkylimidazole, insbesondere N-C₁- bis C₄-Alkylimidazole zur Deprotonierung der Thiazoliumsalze eingesetzt werden. Des weiteren können anorganische Basen wie Alkalimetall- und Erdalkalimetallhydrogencarbonate oder Alkalimetall- und Erdalkalimetallcarbonate zur Erzeugung der Thiazoliumylidkatalysatoren verwendet werden. Die Verwendung von Alkalimetall- und Erdalkalimetallhydroxid-Lösungen ist ebenfalls möglich. Vorteilhaft lassen sich auch Lösungen der basisch reagierenden Alkalimetallcarboxylate einsetzen, wobei im allgemeinen die Natrium- und Kaliumsalze von C₁- bis C₄-Carbonsäuren bevorzugt sind.

Die Thiazoliumylidkatalysatoren können aus den genannten Vorläuferverbindungen nach den genannten Methoden in situ im erfindungsgemmäß angewandten, wäßrig-organischen 2-Phasensystem erzeugt werden, es kann aber auch vorteilhaft sein, die Thiazoliumylidkatalysatoren in einer separaten Umsetzung nach den genannten Methoden zu erzeugen, sie zu isolieren und die derart isolierten, reinen Thiazoliumylidkatalysatoren als solche dem erfindungsgemäßen Reaktionssystem zuzusetzen. Ein Verfahren zur Gewinnung der reinen Thiazoliumylid-Katalysatoren ist in der deutschen Patentanmeldung Anmeldenummer P 41 22 669.0 beschrieben, auf die hiermit Bezug genommen wird.

Das erfindungsgemäße Verfahren kann in einem weiten Temperaturbereich ausgeübt werden, beispielsweise bei Temperaturen von 20 bis 160^{o}C, vorzugsweise bei 60 bis 150^{o}C und besonders bevorzugt bei 100 bis 150^{o}C. Dabei wird zweckmäßigerweise unter dem Eigendruck des angewandten Reaktionssystems gearbeitet.

Der pH-Wert der wäßrigen Phase wird im allgemeinen auf einen pH-Wert von weniger als 8, vorzugsweise auf einen pH-Wert von 2 bis 7, insbesondere von 3 bis 6 eingestellt und während der Umsetzung in diesem Bereich gehalten. Das Arbeiten in diesen pH-Bereichen hat bei der erfindungsgemäßen Durchführung der DHA-Herstellung im 2-Phasen-System den Vorteil, daß Thiazoliumylid-Katalysatoren gegenüber einem Angriff von Wasser auf ihr katalytisch aktives Zentrum stabil sind. Unerwarteterweise bleibt die hohe katalytische Aktivität der eingesetzten Thiazoliumylid-Katalysatoren auch bei diesen niederen pH-Werten erhalten, so daß die Katalysatoren in ihrer Aktivität auch durch acide Nebenprodukte der Umsetzung, beispielsweise Carbonsäuren, nicht beeinträchtigt werden. Es ist also bei Anwendung des erfindungsgemäßen Verfahrens möglich und vorteilhaft, die Kondensation von Formaldehyd zu DHA im sauren pH-Bereich durchzuführen.

### Beispiele

### Beispiel 1

In einem Druckglasgefäß wurden 10,06 g einer 37 gew.-%igen, wäßrigen Formaldehydlösung (Formalin®), 10,2 g Dodecanol und 1,099 g N-Hexadecylbenzothiazoliumylid vorgelegt und anschließend 30 Minuten lang bei 140^{o}C gerührt. Nach Abkühlung der Reaktionsmischung auf Raumtemperatur enthielt die wäßrige Phase 30,4 Gew.-% DHA, entsprechend einer Ausbeute von 82 %.

### Beispiele 2 bis 4

Die Beispiele 2 bis 4 zeigen die Durchführbarkeit des erfindungsgemäßen Verfahrens mit verschiedenerlei Thiazoliumylid-Katalysatoren.
Ansatz: 4,86 g (60 mmol) Formalin® (37 %ig).
5,0 g 2-Ethylhexanool
0,155 g (1,5 mmol) Triethylamin und
1,5 mmol Katalysator A, B oder C.

Zur genaueren Dosierung des Triethylamins wurden 50 g Formalin® mit 1,55 g Triethylamin versetzt und davon 5,0 g sofort verwendet. Die verschiedenen Ansätze wurden bei 100°C jeweils 1 h lang intensiv gerührt. Nach jeweils 15, 30 und 60 Minuten wurde der wäßrigen Phase eine Probe entnommen und deren DHA-Gehalt mittels Gaschromatographie (GC) bestimmt. Die Ergebnisse der Beispiele 2 bis 4 sind in der folgenden Tabelle I aufgelistet.

**Tabelle I**

| Bsp. Nr. | Katalysator^{*)} | Temperatur (⁰C) | Zeit (min) | DHA (Gew.-% in Lösung) | Ausbeute (%) |
|---|---|---|---|---|---|
| 2 | A | 100 | 15 | 4.00 | 10.8 |
| | A | 100 | 30 | 4.96 | 13.4 |
| | A | 100 | 60 | 7.04 | 19.0 |
| 3 | B | 100 | 15 | 0.74 | 2.0 |
| | B | 100 | 30 | 1.34 | 3.6 |
| | B | 100 | 60 | 2.54 | 6.9 |
| 4 | C | 100 | 15 | 18.44 | 49.8 |
| | C | 100 | 30 | 28.81 | 77.9 |
| | C | 100 | 60 | 26.92 | 72.7 |

| | | | | | |
|---|---|---|---|---|---|
| ^{*)} A = N-Hexadecylthiazoliumbromid B = N-Hexadecylbenzothiazoliumbromid C = N-Hexadecylnaphthathiazoliumbromid | | | | | |

### Beispiele 5 bis 14

Die Beispiele 5 bis 14 zeigen die Verwendbarkeit unterschiedlicher Lösungsmittel im erfindungsgemäßen Verfahren.

Ein Ansatz aus 4,86 (60 mmol) Formalin® (37 %ig), 5,0 g Lösungsmittel, 0,155 g (1,5 mmol) Triethylamin und 0,585 g N-Hexadecylthiazoliumbromid wurden 30 Minuten bei 100^{o}C intensiv gerührt. Nach beendeter Umsetzung wurde der DHA-Gehalt der wäßrigen Phase mittels HPLC bestimmt. Die Ergebnisse dieser Versuchsreihe sind in Tabelle II aufgeführt.

**Tabelle II**

| Bsp. Nr. | Lösungsmittel | Zeit (Minuten) | Dihydroxyaceton (Gew.-% in Lösung) |
|---|---|---|---|
| 5 | n-Hexanol | 30 | 1.70 |
| 6 | n-Octanol | 30 | 3.38 |
| 7 | n-Decanol | 30 | 4.62 |
| 8 | n-Dodecanol | 30 | 5.23 |
| 9 | n-Hexadecanol | 30 | 6.54 |
| 10 | Cyclohexanol | 30 | 1.06 |
| 11 | Dodecandiol-1,12 | 30 | 1.01 |
| 12 | 2-Ethylhexanol | 30 | 4.96 |
| 13 | Dibutylformamid | 30 | 2.52 |
| 14 | Dibutylformamid/n-Octanol 1:1 | 30 | 3.55 |

### Beispiele 15 bis 21

Diese Versuchsserie belegt, daß das als Katalysator eingesetzte Thiazoliumsalz im erfindungsgemäßen Verfahren durch ein breites Spektrum von organischen und anorganischen Basen aktiviert, d.h. in das katalytisch aktive Thiazoliumylid umgewandelt werden kann. Insbesondere muß hervorgehoben werden, daß auch schwache Basen, wie Pyridin, unter den angewandten Phasentransferbedingungen in der Lage sind, den Katalysator zu deprotonieren und somit zu aktivieren, was unter homogenen Reaktionsbedingungen nicht möglich ist.

Ein Ansatz aus 4,86 g (60 mmol) Formalin®, 5,0 g Ethylhexanol, 1,5 mmol Base und 0,585 (1,5 mmol) N-Hexadecylthiazoliumbromid wurde 30 Minuten bei 100^{o}C intensiv gerührt. Danach wurde der DHA-Gehalt der wäßrigen Phase mittels GC bestimmt. Die Ergebnisse dieser Versuchsreihe befinden sind in Tabelle III.

**Tabelle III**

| Bsp. Nr. | Base | Reaktionszeit (Minuten) | DHA (Gew.-% in der Lösung) |
|---|---|---|---|
| 15 | NaHCO₃ | 30 | 5.23 |
| 16 | Na₂CO₃ | 30 | 3.02 |
| 17 | Natriumacetat | 30 | 13.41 |
| 18 | Natriumformiat | 30 | 7.28 |
| 19 | Pyridin | 30 | 3.25 |
| 20 | N-Methylimidazol | 30 | 7.43 |
| 21 | NaOH | 30 | 4.99 |

## Patentansprüche

1. Verfahren zur Herstellung von Dihydroxyaceton durch die Thiazoliumylid-katalysierte Kondensation von Formaldehyd, dadurch gekennzeichnet, daß man die Umsetzung in einem Reaktionssystem bestehend aus einer wäßrigen und mindestens einer organischen, flüssigen Phase und bei einem pH-Wert der wäßrigen Phase von weniger als 8 ausführt.

2. Verfahren nach Anspruch 1, dadurch gekennzeichnet, daß man eine wäßrige Lösung von Formaldehyd als Ausgangsmaterial einsetzt.

3. Verfahren nach Anspruch 1, dadurch gekennzeichnet, daß man einen Alkohol mit mehr als 4 Kohlenstoffatomen oder ein Gemisch eines nicht mit Wasser mischbaren, organischen Lösungsmittels mit einem solchen Alkohol als organische Phase verwendet.

4. Verfahren nach Anspruch 1, dadurch gekennzeichnet, daß man die Umsetzung bei 20 bis 180°C durchführt.

5. Verfahren nach Anspruch 1, dadurch gekennzeichnet, daß man lipophile Thiazoliumylide als Katalysatoren verwendet.

6. Verfahren gemäß Anspruch 1, dadurch gekennzeichnet, daß man Naphthathiazoliumylide als Katalysatoren benutzt.

7. Verfahren nach Anspruch 1, dadurch gekennzeichnet, daß man die Umsetzung in einem pH-Bereich von 3 bis 7 durchführt.

## Claims

1. A process for preparing dihydroxyacetone by condensation, catalyzed by a thiazolium ylide, of formaldehyde, wherein the reaction is carried out in a system composed of an aqueous phase and at least one organic liquid phase and at a pH of the aqueous phase of less than 8.

2. A process as claimed in claim 1, wherein an aqueous solution of formaldehyde is used as starting material.

3. A process as claimed in claim 1, wherein an alcohol with more than 4 carbons, or a mixture of an alcohol of this type with an organic solvent which is immiscible with water is used as organic phase.

4. A process as claimed in claim 1, wherein the reaction is carried out at from 20 to 180°C.

5. A process as claimed in claim 1, wherein lipophilic thiazolium ylides are used as catalysts.

6. A process as claimed in claim 1, wherein naphthothiazolium ylides are used as catalysts.

7. A process as claimed in claim 1, wherein the reaction is carried out at a pH of from 3 to 7.

## Revendications

1. Procédé de préparation de dihydroxyacétone par la condensation de formaldéhyde catalysée par un ylure de thiazolium, caractérisé en ce que l'on conduit la réaction dans un système réactionnel se composant d'une phase aqueuse et d'au moins une phase organique liquide et à un pH de la phase aqueuse de moins de 8.

2. Procédé selon la revendication 1, caractérisé en ce que l'on utilise, comme matière de départ, une solution aqueuse de formaldéhyde.

3. Procédé selon la revendication 1, caractérisé en ce que l'on utilise, comme phase organique, un alcool à plus de 4 atomes de carbone ou un mélange d'un solvant organique non miscible à l'eau avec un tel alcool.

4. Procédé selon la revendication 1, caractérisé en ce que l'on mène la réaction à une température de 20 à 180°C.

5. Procédé selon la revendication 1, caractérisé en ce que l'on utilise, comme catalyseurs, des ylures de thiazolium lipophiles.

6. Procédé selon la revendication 1, caractérisé en ce que l'on utilise, comme catalyseurs, des ylures de naphtathiazolium.

7. Procédé selon la revendication 1, caractérisé en ce que l'on mène la réaction dans une gamme de pH de 3 à 7.
